(19) Europäisches Patentamt
European Patent Office
Office européen des brevets

(11) Publication number: **0 143 628**
**B1**

(12) **EUROPEAN PATENT SPECIFICATION**

(45) Date of publication of patent specification: **11.10.89**

(51) Int. Cl.$^4$: **C 07 C 102/04,** C 07 C 103/76

(21) Application number: **84308164.7**

(22) Date of filing: **26.11.84**

(54) **Process for preparing salicylamide compounds.**

(30) Priority: **28.11.83 US 555760**

(43) Date of publication of application:
**05.06.85 Bulletin 85/23**

(45) Publication of the grant of the patent:
**11.10.89 Bulletin 89/41**

(84) Designated Contracting States:
**AT BE CH DE FR GB IT LI NL SE**

(56) References cited:
**EP-A-0 038 191**
**EP-A-0 038 192**

**ORGANIC SYNTHESES, vol. 26, 1946, pages 92-94; C.F.H. ALLEN et al.: "Salicyl-o-toluide"**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(72) Inventor: **Schwarz, Joshua**
**1557 East 18th Street**
**Brooklyn, County of Kings New York (US)**

(74) Representative: **van Gent, Jan Paulus et al**
**Unilever N.V. Patent Division P.O. Box 137**
**NL-3130 AC Vlaardingen (NL)**

**EP 0 143 628 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a new and improved process for the preparation of salicylamide compounds.

Salicylamide compounds, especially those bearing n-alkyl or n-alkanoyl substituents in the benzene ring of the salicylic acid portion thereof, are described in US Patents Nos. 4 358 433 and 4 287 191 which disclose the use of such compounds as bactericides and as anti-dental plaque agents.

The synthesis of a typical unsubstituted salicylamide is conventionally conducted by the reaction of salicyclic acid and aniline in the presence of phosphorus trichloride as a condensing agent. US Patents Nos 2 763 683; 3 221 051; 3 221 052 and 3 231 611 describe various processes for the preparation and purification of salicylanilide. However, while the processes described therein are presumably quite effective for the preparation and purification of the parent salicylanilide, they are not practical for the preparation of salicylanilides substituted in the salicyl and/or aniline portions of the molecule or other salicylamide compounds wherein the aniline portion of the molecule is wholly replaced by a substituted or unsubstituted heterocyclic amine.

There is another method for the preparation of salicylamides known as the salol reaction. Salol is a common name for phenyl salicylate and the salol process involves the formation of amides of salicylic acid by the heating of phenyl salicylate with an amine which may be an aniline or a heterocyclic amine. The earliest report relative to the salol process appears to be by M. Schopff, *Ber. 25,* 2740 (1892). A concise description of the salol process in the English language is contained in the Merck Index, 8th Edition, page 1211 together with a listing of various other publications in which the salol reaction has been mentioned or discussed.

According to the salol process, a given quantity of phenyl salicylate is heated with at least a stoichiometric corresponding quantity of aniline, usually in a high boiling aromatic diluent at temperatures approaching and even exceeding 200°C for several hours. The resulting reaction product is thereafter allowed to crystallise, washed with ligroin and optionally recrystallised from ethanol. The aforementioned process has been described with reference to the preparation of both the parent salicylanilide as well as several salicylanilides where the substitution has been in the benzene ring of the aniline portion alone.

According to a report by C F H Allen et al in "Organic Syntheses, Collective Volume III", 765 (1955), when the parent salicylanilide compound is synthesised the yield is only about 70% and moreover, the resulting product has a persistent pink colour which is not easily removed. While Allen et al do report higher yields in the syntheses of other salicylamides, it is interesting to note that the only variation from the basic salol process synthesis of salicylanilide suggested therein is one involving the use of different anilines and heterocyclic amines. There is no suggestion made therein with respect to the usefulness or otherwise of the salol process in those cases where the phenyl salicylate reactant itself incorporates a substituent or substituents upon its salicyclic acid benzene ring.

Due consideration of mechanistic concepts enables one to conclude that substitution of the benzene ring of the salicylic acid portion of the phenyl salicylate molecule with an alkanoyl group would led to considerably more complexity in the reaction pathway as a result of the complexation of the aniline or other amine wth the alkanoyl substituent group. The result would be lower yields of the desired product, increased side reactions and more difficulty in purifying the desired salicylamide product.

It is an object of this invention to provide a new and improved process for the preparation in high yield and purity of salicylamides of the formula:

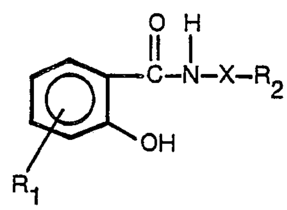

Formula I

where

X is a benzene ring or a heterocyclic aromatic group

$R_1$ is —$COC_nH_{2n+1}$ where n is an integer of from 1 to 15, and

$R_2$ is a hydrogen atom or a substituent group which is CN, $NO_2$, F, Cl, Br, I, $CF_3$, $CCl_3$, $CBr_3$, $CI_3$, $COCH_nH_{2n+1}$ or $C_nH_{2n+1}$.

According to the present invention there is provided a process for the preparation of salicylamides of Formula I which comprises the steps of:—

(a) reacting one mole part of a phenyl salicylate ester bearing an $R_1$ substituent on the benzene ring of the salicylic acid portion thereof with about one to two mole parts of an amine $NH_2$—X—$R_2$ at a temperature of above about 150°C, preferably between about 150°C and about 225°C, for about 2 to about 6 hours to form a reaction mixture;

(b) dissolving the reaction mixture in a solvent which is a polar organic solvent or a halogenated hydrocarbon or mixture thereof to form a dissolved reaction mixture;

(c) acidifying the dissolved reaction mixture with an aqueous solution of a Lowry-Bronsted acid (eg. hyrochloric acid) to form an acidified dissolved reaction mixture which preferably has a pH below about 9.0, more desirably below about 6.0;

(d) refluxing the acidified dissolved reaction mixture to produce a final reaction mixture;

(e) adding water to the final reaction mixture to precipitate the product salicylamide compound; and

(f) recovering the precipitated product salicylamide compound from the final reaction mixture.

In step (a) the mole ratio of amine to ester is preferably about 1:1 to 1.5:1 and more preferably about 1.2:1 to 1.3:1. The mole ratio selected is based mainly on economics. The most expensive reactant is the salicylate ester and, accordingly, it is desired to use a sufficient excess of the amine reactant to react completely with the ester. Large excesses of the amine reactant are undesirable because of Schiff base side product formation by reaction of the amine with the carbonyl group of the side chain. The discovery of this side product formation led to refluxing the acidified reaction mixture in step (d) in order to hydrolyse any Schiff base present and thereby recover additional desired product.

The reaction between the phenyl salicylate ester and the amine $NH_2$—X—$R_2$ may be conducted in the presence of an inert solvent system such as a halogenated or unhalogenated aromatic compound or a polyethylene glycol of average molecular weight about 1000 to about 6000 or mixtures thereof of all having a melting point of up to 120°C.

The solvent employed in step (b) may be an alkanol, halogenate hydrocarbon or mixture thereof all having a boiling point of up to 200°C, eg ethanol or methylene chloride. The step of dissolving the reaction mixture produced in step (a) in the solvent may optionally be conducted after first cooling the reaction mixture to a temperature of about 120°C to about 40°C.

In step (d) the refluxing of the acidified dissolved reaction mixture is performed at a sufficiently high temperature to form the product for a few minutes up to about one hour or even longer to produce a final reaction mixture. Longer times may be used but could result in undesirable hydrolysis of the product. Preferably, reflux will be from about 15 to 30 minutes to minimise undesirable hydrolysis. The reflux temperature can vary widely depending on the solvent used, eg. methanol, ethanol, isopropanol, n-propanol, and the like. When ethanol is used, the mixture refluxes at about 80°C. It is possible to use temperatures between about 40°C and 120°C or even higher if the reflux were operated under pressure. The lower the temperature the longer the heating time required while higher temperatures require shorter heating times compared to the fifteen minutes preferably used when the temperature is 80°C.

The product salicylamide compound obtained by the above process may, if desired, be purified by recrystallisation from, for example, ethanol or an ethanol/water mixture. Any other solvent in which the product salicylamide dissolves may be gainfully employed for the foregoing purpose. The choice of a suitable solvent will be a matter of obvious alternatives to a person of ordinary skill in the art to which the invention relates.

Examples of heterocyclic amines of the formula $NH_2$—X—$R_2$ which may be used as a reactant in step (a) of the process of this invention are 2-aminofuran, 2-aminothiazole, 2-aminobenzothiazole and 8-aminopurine. The group X may be comprised of other monocyclic or fused ring polycyclic or non-fused ring polycyclic heterocyclic aromatic groups if desired.

In a preferred aspect the process of the invention is applied to the preparation of salicylamides of the formula:

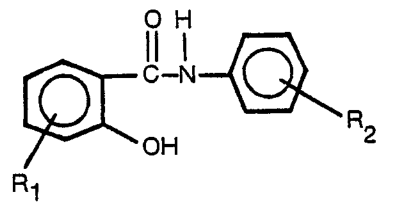

wherein $R_1$ and $R_2$ have the above meanings.

The following Examples, which are submitted for illustrative purposes only, demonstrate the synthesis of a representative salicylamide compound, ie., 3′-trifluoromethyl-5-octanoylsalicylanilide with remarkably high yields and exceptionally high purity. The purity of the salicylamide reaction product was determined in each case using high performance liquid chromatography, as well as standard spectroscopic methods, including nuclear magnetic resonance spectral analyses.

## Example 1

50 g (0.147 mole) 5-octanoyl phenyl salicylate and 29.6 g (0.184 mole) meta-trifluoromethylaniline were placed in a reaction vessel under a blanket of nitrogen. The mixture was heated to 180° for 3.5 hours. The temperature was lowered to about 100°C and about 80 ml of ethanol was added to dissolve the reaction mixture. About 10 ml of concentrated hydrochloric acid (37%) was added to 10 ml of water and then added slowly to the reaction vessel with vigorous stirring. The reaction mixture was refluxed at about 80°C for about 15 minutes. 25 ml of water was then added with vigorous stirring whereupon a yellow solid product precipitated out. After drying, the solid produce was stirred with about 225 ml petroleum ether and filtered. This resulted in 42.5 g of the product, ie. 3′-trifluoromethyl-5-octanoylsalicylanilide. The product was dissolved in sufficient ethanol to effect dissolution and water was thereafter added dropwise until the cloud point of the solution was reached. Thereafter, the purified product was precipitated by cooling the solution. Recrystallisation as aforesaid from said enthanol/water mixture yielded 38.2 g (0.113 mole) of product with purity greater than 99.5%.

It will be noted that the product yield obtained was 76.9% upon the basis of the 5-octanoyl phenyl salicylate reactant.

## Example 2

50 g (0.147 mole) 5-octanoyl phenyl salicylate and 29.8 g (0.185 mole) meta-trifluoromethylaniline were placed in a reaction vessel under a blanket of nitrogen. The mixture was heated to 150—155°C for 6 hours. The temperature was

lowered to about 100°C and about 80 ml of ethanol was added to dissolve the reaction mixture. About 10 ml of concentrated hydrochloric acid (37%) was added to 10 ml of water and then added slowly to the reaction vessel with vigorous stirring. The reaction mixture was refluxed at about 80°C for about 15 minutes. 25 ml of water was then added with vigorous stirring whereupon a yellow solid product precipitated out. The solid product was recovered by filtration and washed with water. After drying, the solid product was stirred with about 225 ml petroleum ether and again filtered. This resulted in 41.9 g (0.103 mole) of the product, ie. 3′-trifluoromethyl-5-octanoylsalicylanilide. Recrystallisation from an ethanol/water mixture in accordance with the procedure of Example 1 yielded 35.9 g (0.088 mole) of product with purity greater than 99.5%.

It will be noted that the initial product yield obtained was 70.1% upon the basis of the 5-octanoyl phenyl salicylate reactant. In this particular case, it will also be noted that the net yield of the product salicylamide was 59.9% upon the basis of the precursor 5-octanoyl phenyl salicylate followinig the recrystallisation of the initially obtained product from an ethanol/water mixture.

Example 3

50 g (0.147 mole) 5-octanoyl phenyl salicylate and 29.8 g (0.185 mole) meta-trifluoromethylaniline were placed in a reaction vessel under a blanket of nitrogen. The mixture was heated to 200—205°C for 2 hours. The temperature was lowered to about 100°C and about 80 ml of ethanol was added to dissolve the reaction mixture. About 10 ml of concentrated hydrochloric acid (37%) was added to 10 ml of water and then added slowly to the reaction vessel with vigorous stirring. The reaction mixture was refluxed at about 80°C for about 15 minutes. 25 ml of water was then added with vigorous stirring whereupon a yellow solid product precipitated out. The solid product was recovered by filtration and washed with water. After drying, the solid product was stirred with about 225 ml petroleum ether and again filtered. This resulted in 42.5 g of the product, ie. 3′-trifluoromethyl-5-octanyolsalicylanilide. Recrystallisation from an ethanol/water mixture in accordance with the procedure of Example 1 yielded 38.2 g (0.113 mole) of product with purity greater than 99.5%.

It will be noted that the product yield obtained was 76.9% upon the basis of the 5-octanoyl phenyl salicylate reactant.

Example 4

20 g (0.058 mole) of 5-octanoyl phenyl salicylate and 11.9 g (0.073 mole) of meta-trifluoromethylaniline were placed in a reaction vessel with 45 ml of 1,2,4-trichlorobenzene under a blanket of nitrogen. The mixture was heated to 225°C for 4 hours. It was then allowed to cool to about 40°C at which time 20 ml of methylene chloride was added to avoid solidification of the reaction mixture. Upon further cooling in an ice-water bath, a yellow solid product precipitated out and was recovered by filtration. About 5 ml of concentrated hydrochloric acid (37%) was added to 5 ml of water and 30 ml of ethanol in a flask. The yellow solid product was added to the flask with vigorous stirring and the dissolved mixture was refluxed at about 80°C for about 15 minutes. 15 ml of water was then added thereto with vigorous stirring whereupon a slightly yellowish solid precipitated out. The precipitate was recovered by filtration, dried and recrystallised from ethanol to yield 17 g (0.042 mole) of the pure product, ie. 3′-trifluoromethy-5-octanoylsalicylanilide.

It will be noted that the product yield obtained was 72.4% upon the basis of the 5-octanoyl phenyl salicylate reactant.

**Claims**

1. A process for preparing a salicylamide compound of the formula:

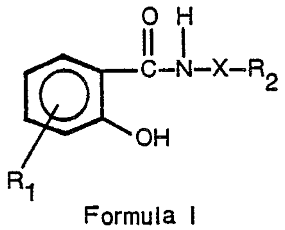

Formula I

where

X is a benzene ring or a heterocyclic aromatic group

$R_1$ is $—COC_nH_{2n+1}$ where n is an integer of from 1 to 15, and

$R_2$ is a hydrogen atom or a substituent group which is CN, $NO_2$, F, Cl, Br, I, $CF_3$, $CCl_3$, $CBr_3$, $CI_3$, $COCH_nH_{2n+1}$ or $C_nH_{2n+1}$

which process comprises the steps of

(a) reacting one mole part of a phenyl salicylate ester optionally bearing an $R_1$ substituent group on the benzene ring of the salicylic acid portion thereof with about one to two mole parts of an amine $NH_2—X—R_2$ at a temperature of above about 150°C for about 2 to 6 hours to form a reaction mixture;

(b) dissolving the reaction mixture in a solvent which is a polar organic solvent or a halogenated hydrocarbon or mixture thereof to form a dissolved reaction mixture;

(c) acidifying the dissolved reaction mixture with an aqueous solution of a Lowry-Bronsted acid to form an acidified dissolved reaction mixture;

(d) refluxing the acidified dissolved reaction mixture to produce a final reaction mixture;

(e) adding water to the final reaction mixture to precipitate the product salicylamide compound; and

(f) recovering the precipitated product salicylamide compound from the final reaction mixture.

2. A process as claimed in Claim 1 wherein the phenyl salicylate ester and the amine are reacted at a temperature in the range of about 150°C to about 225°C.

3. A process as claimed in Claim 1 or Claim 2 wherein the reaction mixture is cooled to a temperature in the range of about 120°C to about 40°C prior to the addition of said solvent.

4. A process as claimed in any of Claims 1 to 3 wherein said solvent is an alkanol having a boiling point of up to 200°C, a halogenated alkane having a boiling point of up to 200°C or a mixture thereof.

5. A process as claimed in any of Claims 1 to 4 wherein the Lowry-Bronsted acid is hydrochlorid acid.

6. A process as claimed in any of Claims 1 to 5 wherein the phenyl salicylate ester and the amine are reacted in the presence of an organic solvent system consisting of a halogenated aromatic compound having a melting point of up to 120°C, an unhalogenated aromatic compound having a melting point of up to 120°C, a polyethylene glycol having an average molecular weight of about 1000 to about 6000 or a mixture thereof.

7. A process as claimed in any of Claims 1 to 6 wherein the amine $NH_2—X—R_2$ is an aniline or an $R_2$-substituted aniline.

**Patentansprüche**

1. Verfahren zur Herstellung eines Salicylamids der Formel:

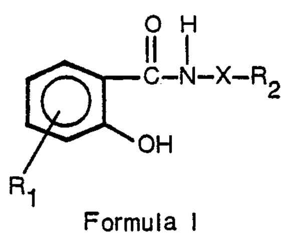

Formula I

worin

X ein Benzolring oder eine heterozyklische aromatische Gruppe ist

$R_1$ $—COC_nH_{2n+1}$, worin n eine ganze Zahl von 1 bis 15 darstellt, ist und

$R_2$ ein Wasserstoffatom oder eine substituierte Gruppe ausgewählt unter CN, $NO_2$, F, Cl, Br, I, $CF_3$, $CCl_3$, $CBr_3$, $Cl_3$, $COC_nH_{2n+1}$ und $C_nH_{2n+1}$ ist,

wobei das Verfahren folgende Schritte umfaßt:

(a) Umsetzung eines Molanteils eines Phenylsalicylatesters mit gegebenenfalls einer $R_1$-Substituenten-Gruppe an dem Benzolring des Salicylsäureanteils davon mit etwa eins bis zwei Molanteilen eines Amins $NH_2—X—R_2$ bei einer Temperatur oberhalb etwa 150°C während etwa 2 bis 6 Stunden zur Bildung einer Reaktionsmischung;

(b) Auflösen der Reaktionsmischung in einem Lösungsmittel, welches ein polares organisches Lösungsmittel oder ein halogenierter Kohlenwasserstoff oder eine Mischung davon ist, zur Bildung einer gelösten Reaktionsmischung;

(c) Ansäuern der gelösten Reaktionsmischung mit einer wäßrigen Lösung einer Lowry-Bronsted-Säure zur Bildung einer angesäuerten gelösten Reaktionsmischung;

(d) Rückflußkochen der angesäuerten gelösten Reaktionsmischung zur Herstellung einer abschließenden Reaktionsmischung;

(e) Zusetzen von Wasser zu der abschließenden Reaktionsmischung zur Ausfällung des Salicylamid-Produktes; und

(f) Gewinnung des ausgefällten Salicylamid-Produktes aus der abschließenden Reaktionsmischung.

2. Verfahren nach Anspruch 1, worin der Phenylsalicylatester und das Amin umgesetzt werden bei einer Temperatur im Bereich von etwa 150°C bis etwa 225°C.

3. Verfahren nach Anspruch 1 oder 2, worin die Reaktionsmischung gekühlt wird auf eine Temperatur im Bereich von etwa 120°C bis etwa 40°C vor der Zugabe des Lösungsmittels.

4. Verfahren nach einem der Ansprüche 1 bis 3, worin das Lösungsmittel ein Alkanol mit einem Siedepunkt von bis zu 200°C, ein halogeniertes Alkan mit einem Siedepunkt von bis zu 200°C oder eine Mischung davon ist.

5. Verfahren nach einem der Ansprüche 1 bis 4, worin die Lowry-Bronsted-Säure Salzsäure ist.

6. Verfahren nach einem der Ansprüche 1 bis 5, worin der Phenylsalicylatester und das Amin umgesetzt werden in Anwesenheit eines organischen Lösungsmittelsystems, bestehend aus einer halogenierten aromatischen Verbindung mit einem Schmelzpunkt von bis zu 120°C, einer nicht-halogenierten aromatischen Verbindung mit einem Schmelzpunkt von bis zu 120°C, einem Polyethylenglykol mit einem durchschnittlichen Molekulargewicht von etwa 1000 bis etwa 6000 oder einer Mischung davon.

7. Verfahren nach einem der Ansprüche 1 bis 6, worin das Amin $NH_2—X—R_2$ Anilin oder ein $R_2$-substituiertes Anilin ist.

**Revendications**

1. Procédé de préparation d'un salicylamide de formule:

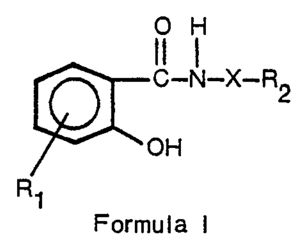

Formula I

dan laquelle X est un noyau benzénique ou un groupe aromatique hétérocyclique, $R_1$ représente le groupe $—COC_nH_{2n+1}$ dans lequel n est un nombre entier de 1 à 15, et $R_2$ est un atome d'hydrogène ou un groupe substituant qui est CN, $NO_2$, F, Cl, Br, I, $CF_3$, $CCl_3$, $CBr_3$, $Cl_3$, $COC_nH_{2n+1}$ ou $C_nH_{2n+1}$, procédé qui consiste:

(a) à faire réagir 1 mole partie d'un salicylate de phényle portant facultativement un groupe substituant $R_1$ sur le noyau benzénique de la partie acide salicylique de celui-ci, avec environ 1 à 2 moles parties d'une amine $NH_2—X—R_2$ à une température supérieure à environ 150°C pendant environ 2 à 6 heures pour former un mélange de réaction;

(b) à dissoudre le mélange de réaction dans un solvant qui est un solvant organique polaire ou un hydrocarbure halogéné ou un mélange de ceux-ci pour former un mélange de réaction dissous;

(c) à acidifier le mélange de réaction dissous avec une solution aqueuse d'un acide Lowry-Bronsted pour former un mélange de réaction dissous acidifié;

(d) à porter au reflux le mélange de réaction disous acidifié pour produire un mélange de réaction final;

(e) à ajouter de l'eau au mélange de réaction final pour précipiter le produit salicylamide; et

(f) à récupérer le salicylamide précipité du mélange de réaction final.

2. Procédé selon la revendication 1, dans lequel on fait réagir le salicylate de phényle et l'amine à une température comprise entre environ 150 et environ 225°C.

3. Procédé selon la revendication 1 ou 2, dans lequel on refroidit le mélange de réaction à une température comprise entre environ 120 et 40°C avant l'addition du dit solvant.

4. Procédé selon l'une quelconque des revendications 1 à 3, dans lequel ledit solvant est un alcanol ayant un point d'ébullition pouvant aller jusqu'à 200°C, un alcane halogéné ayant un point d'ébullition pouvant aller jusqu'à 200°C ou un mélange de ceux-ci.

5. Procédé selon l'une quelconque des revendications 1 à 4, dans lequel l'acide Lowry-Bronsted est l'acide chlorhydrique.

6. Procédé selon l'une quelconque des revendicaitons 1 à 5, dans lequel le salicylate de phényle et l'amine sont mis à réagir en présence d'un système solvant organique consistant en un composé aromatique halogéné ayant un point de fusion jusqu'à 120°C, un composé aromatique non halogéné ayant un point de fusion jusqu'à 120°C, un polyéthylène-glycol ayant une masse moléculaire moyenne d'environ 1000 à 6000 ou un mélange de ceux-ci.

7. Procédé selon l'une quelconque des revendications 1 à 6, dans lequel l'amine $NH_2—X—R_2$ est l'aniline our une aniline substituée par $R_2$.